# EUROPEAN PATENT APPLICATION

(11) **EP 1 612 262 A1**
(43) Date of publication of application: **04.01.2006**
(21) Application number: 04726636.6
(22) Date of filing: 08.04.2004
(51) Int. Cl.: C12M 3/00, H02J 9/00

(54) **CULTURE TREATING DEVICE AND AUTOMATIC CULTURE TREATING DEVICE**

(30) Priority: 09.04.2003 JP 2003105316; 30.05.2003 JP 2003154735
(71) Applicant: Olympus Corporation, Tokyo 151-0072 (JP)
(72) Inventor: HIBINO, Hiroki, Hachioji-shi, Tokyo 193-0811 (JP); FUKUDA, Hiroshi, Hino-shi, Tokyo 191-0053 (JP); NUMATA, Kiminobu, Hachioji-shi, Tokyo 192-0045 (JP); MACHIDA, Hiroyuki, Hachioji-shi, Tokyo 192-0916 (JP); KINOSHITA, Tomoyuki, Hachioji-shi, Tokyo 192-0023 (JP); NAKAMURA, Kenji, Hachioji-shi Tokyo 192-0045 (JP)
(74) Representative: von Hellfeld, Axel
(86) International application number: PCT/JP2004/005057
(87) International publication number: WO 2004/090093

(57) **Abstract**

There are provided a culture treatment apparatus and an automatic culture apparatus capable of taking quick and appropriate action even if there is a change in the state of a space where cells stored in a container, such as a culture vessel, for the automatic culture apparatus are subjected to predetermined treatment. The culture treatment apparatus includes a treatment section for applying predetermined treatment to cells stored in an openable container for the automatic culture apparatus in a partitioned space; a detection section for detecting a predetermined state of the space, the detection section being in the space or in the vicinity of the space; and a control section for controlling the treatment section to close the container if the detection section detects the predetermined state with the container opened.

## Description

### Technical Field

The present invention relates to an automatic culture apparatus for automatically carrying out cell culturing outside a living organism.

### Background Art

Regenerative medicine for culturing cells outside a living organism and rebuilding tissues to use cultured tissue equivalents for therapy is gaining attention. A known automatic culture apparatus for culturing such cells includes an incubator provided with a stationary storage rack (stacker) that can accommodate a plurality of culture vessels and a horizontally, vertically, and rotationally transferable transfer mechanism that can transfer the culture vessels; a culture-medium replacing robot provided with a culture-medium dispensing needle and a culture-medium discharging needle; and a management computer for comprehensively controlling these components (see for example, Japanese Unexamined Patent Application Publication No. 2002-262856 (e.g., Fig. 1)).

This automatic culture apparatus is designed such that a culture vessel is taken out from the incubator with a transfer robot, the lid of the culture vessel is removed with a lid-opening robot, and a culture medium in the culture vessel is discharged and dispensed with the culture-medium replacing robot. After the replacement of the culture medium is completed, the lid of the culture vessel is closed again by the lid-opening robot and the culture vessel is stored in the stacker in the incubator by the transfer robot. The operation of this automatic culture apparatus is automatically carried out by the management computer, which also carries out scheduling management.

In the automatic culture apparatus described in Japanese Unexamined Patent Application Publication No. 2002-262856, the interior of the stacker accommodating culture vessels is managed so that incubation conditions, such as the temperature, the humidity, and the carbon dioxide concentration, are maintained constant. However, the space where the lids of the culture vessels are removed or where the opened culture vessels are subjected to a variety of processing, such as replacing the culture media, is provided outside the stacker. Various mechanical devices such as robots and transfer conveyers are arranged in the space where the cells in the culture vessels are subjected to treatment.

Therefore, this space is an environment where dust readily floats, and it is necessary to reduce the amount of floating dust in this space in some way, otherwise, the problem of dust etc. entering a culture vessel when the lid of the culture vessel is removed will arise.

A space requiring such a degree of cleanliness is typically formed in a clean room where an air-cleaning apparatus, such as filter, is installed.

However, while a culture vessel is subjected to a variety of processing in such a clean room, floating dust in the clean room may increase for some reason, such as because the door of the clean room is opened, to such a degree that exceeds the cleaning power of the air-cleaning apparatus. In this case, dust cannot be completely prevented from entering the culture vessel.

Furthermore, the automatic culture apparatus described in the above-described Japanese Unexamined Patent Application Publication No. 2002-262856 is not provided with an auxiliary power supply, a power-failure circuit, etc. for supplying electrical power in the event of a power failure, and therefore, all operations are stopped, including the management computer, if the power supply is shut down due to, for example, a power failure. For this reason, if the power supply is shut down, for example, while a culture medium is being replaced by the culture-medium replacing robot after the culture vessel is taken out from the stacker with the transfer mechanism, processing of the culture vessel is suspended until the power supply is recovered. In order to avoid this, an operator needs to continuously monitor the automatic culture apparatus.

### Disclosure of Invention

The present invention has been conceived in light of these circumstances, and it is an object of the present invention to provide a culture treatment apparatus and an automatic culture apparatus capable of taking quick and appropriate action even if there is a change in the state of a space where cells stored in a container, such as a culture vessel, in the automatic culture apparatus are subjected to predetermined treatment.

A particular object of the present invention is to provide a culture treatment apparatus and an automatic culture apparatus capable of sufficiently reducing dust etc. entering a container, such as a culture vessel, in the automatic culture apparatus.

It is another object of the present invention to provide a culture treatment apparatus and an automatic culture apparatus that do not require continuous monitoring by an operator even if power is shut down due to, for example, a power failure.

According to a first aspect of the present invention, a culture treatment apparatus includes:
a treatment section for applying predetermined treatment to cells stored in an openable container for an automatic culture apparatus in a partitioned space;
a detection section for detecting a predetermined state of the space, the detection section being in the space or in the vicinity of the space; and
a control section for controlling the treatment section to prevent the container in the space from being opened if the detection section detects the predetermined state.

According to a second aspect of the present invention, a culture treatment apparatus includes:
a treatment section for applying predetermined treatment to cells stored in an openable container for an automatic culture apparatus in a partitioned space;
a detection section for detecting a predetermined state of the space, the detection section being in the space or in the vicinity of the space; and
a control section for controlling the treatment section to close the container if the detection section detects the predetermined state with the container opened.

In the first or second aspect, the detection section may be a cleanliness sensor for detecting whether the degree of cleanliness in the space does not satisfy a predetermined degree of cleanliness.

In a case where the cleanliness sensor is employed as the detection section in the first aspect, the container in the space is prevented from being opened by the operation of the control section if the degree of cleanliness measured with the cleanliness sensor does not satisfy the predetermined degree of cleanliness while the predetermined treatment is performed on the cells in the container for the automatic culture apparatus by the operation of the treatment section. Consequently, the container is prevented from being opened in an environment containing, for example, a large amount of floating dust to reduce the risk of dust etc. being deposited on the cells.

In a case where the cleanliness sensor is employed in the first aspect, the operation of the control section controls the treatment section to close the container for the automatic culture apparatus if the degree of cleanliness measured with this cleanliness sensor does not satisfy the predetermined degree of cleanliness. Therefore, the container is prevented from being left open for an extended period of time in an environment containing, for example, a large amount of dust to reduce the risk of dust etc. being deposited on the cells.

The culture treatment apparatus may be provided with a report section for reporting that the degree of cleanliness measured with the cleanliness sensor does not satisfy the predetermined degree of cleanliness, if the degree of cleanliness measured with the cleanliness sensor does not satisfy the predetermined degree of cleanliness.

By providing the report section, a decrease in the degree of cleanliness in the space where the container for the automatic culture apparatus is disposed is reported through the operation of this report section. Therefore, an operator can recognize this fact to take action sooner.

In the above-described culture treatment apparatus, a plurality of the cleanliness sensors may be arranged at intervals in the space .

When the plurality of cleanliness sensors are arranged, quick action can be taken through the operation of the plurality of cleanliness sensors even when there is a local increase in the amount of floating dust.

The above-described cleanliness sensor may be arranged near a location through which the container passes.

By arranging the above-described cleanliness sensor near a location through which the container passes, the container is allowed to pass through a space whose degree of cleanliness detected by the cleanliness sensor has been judged by the operation of the cleanliness sensor as satisfying the predetermined degree of cleanliness.

If the cleanliness sensor is to be arranged near a location through which the container passes, the cleanliness sensor may be arranged on a mounting stage for holding the container.

If the cleanliness sensor is provided on the mounting stage for holding the container, the degree of cleanliness in the space near the container is always monitored through the operation of this cleanliness sensor. As a result, the container is prevented from being exposed to an environment with a low degree of cleanliness.

The above-described culture treatment apparatus may be provided with a display section for displaying the degree of cleanliness measured with the cleanliness sensor.

By providing the display section, the degree of cleanliness in the space where the container for the automatic culture apparatus is displayed by the operation of this display section. As a result, it is possible to confirm whether the air in the space is clean.

The culture treatment apparatus according to the second aspect may further include an auxiliary power supply for supplying electrical power in the event of a power failure,
wherein the detection section may be a power-failure detecting section for detecting a power failure, and
the control section may switch power supply to the auxiliary power supply if the power-failure detecting section detects a power failure.

In the above-described culture treatment apparatus provided with the auxiliary power supply and the power-failure detecting section, if a power failure occurs while the objects contained in the container for the automatic culture apparatus are subjected to predetermined treatment by the culture treatment apparatus, e.g., while reagents required to culture the contained objects are being dispensed, assuming that the contained objects are cells, after the container for the automatic culture apparatus has been opened by the treatment section, the power-failure control section switches the power supply to the auxiliary power supply based on detection information from the power-failure detecting section. This ensures the supply of power even if a power failure occurs. If the container for the automatic culture apparatus is open, the power-failure control section operates the treatment section to close the container for the automatic culture apparatus. Therefore, the container for the automatic culture apparatus stops in a closed state. In short, if a power failure occurs, the container for the automatic culture apparatus stops in a closed state.

With this arrangement, even if an unpredictable power failure occurs, causing the container for the automatic culture apparatus to be suspended for a certain period of time, an operator does not need to monitor the container for the automatic culture apparatus, unlike before. Therefore, the time and cost required for monitoring can be reduced.

If the predetermined treatment has started when a power failure is detected by the power-failure detecting section, the control section may allow the predetermined treatment to continue until the subsequent closing of the container for the automatic culture apparatus and then stop the culture treatment apparatus.

In this case, even if a power failure occurs during predetermined treatment by the culture treatment apparatus, for example, while reagents are being dispensed to, for example, cells which are the objects contained in the container for the automatic culture apparatus, the power-failure control section allows the predetermined treatment to be continued and then stops the culture treatment apparatus when the container for the automatic culture apparatus is closed. This prevents the culture treatment apparatus from experiencing premature termination of treatment. Furthermore, since this can be accomplished simply by continuing with normal processing, it is not necessary to prepare a separate processing procedure for power failure.

In the above-described culture treatment apparatus provided with the auxiliary power supply and the power-failure detecting section, an open/closed detecting section for detecting an open/closed state of the above-described container for the automatic culture apparatus may be provided.

If the culture treatment apparatus is provided with the open/closed detecting section, this open/closed detecting section detects the open/closed state of the container for the automatic culture apparatus. This ensures that the open/closed state of container for the automatic culture apparatus is detected more accurately to improve the reliability.

In the present invention, the above-described container for the automatic culture apparatus may be at least one of a culture vessel containing cells, a reagent container, and a disposable-tip container.

In the automatic culture apparatus according to the present invention, the container for the automatic culture apparatus is closed when the predetermined state is detected in the space. If the container for the automatic culture apparatus is the culture vessel, dust etc. is prevented from being deposited on cells. If the container for the automatic culture apparatus is the reagent container, dust etc. is prevented from being deposited on reagents. If the container for the automatic culture apparatus is the disposable-tip container, dust etc. is prevented from being deposited on tips.

According to a third aspect of the present invention, an automatic culture apparatus includes:
the culture treatment apparatus described in the first or the second aspect;
an incubation chamber for accommodating the container storing cells such that the container can be extracted and inserted and for culturing the cells while maintaining predetermined incubation conditions; and
a transfer mechanism for transferring the container between the culture treatment apparatus and the incubation chamber.

With the culture treatment apparatus employing a cleanliness sensor as the detection section, the automatic culture apparatus according to the present invention can apply predetermined treatment to the cells stored in the container for the automatic culture apparatus in the space where a high degree of cleanliness is maintained. Furthermore, even if the degree of cleanliness in the space decreases below the predetermined degree of cleanliness, the risk of the cells in the container being contaminated by dust or the like is reduced, which affords an advantage in that stable and effective cell culturing can be realized.

In addition, with the culture treatment apparatus having the auxiliary power supply and the power-failure detecting section, the automatic culture apparatus according to the present invention ensures the supply of power to the culture treatment apparatus even if a power failure occurs. This ensures that the container for the automatic culture apparatus is closed, if opened, when treatment is stopped. With this arrangement, even if an unpredictable power failure occurs, causing the container for the automatic culture apparatus to be suspended for a certain period of time, an operator does not need to monitor the container for the automatic culture apparatus, unlike before. Thus, the time and cost for monitoring can be reduced while still allowing cells to be cultured automatically.

### Brief Description of the Drawings

Fig. 1 is a perspective view of a culture treatment apparatus and an automatic culture apparatus according to one embodiment of the present invention.
Fig. 2 is a schematic longitudinal sectional view of a first space in the automatic culture apparatus shown in Fig. 1.
Fig. 3 is a schematic plan view of the first space in the automatic culture apparatus shown in Fig. 1.
Fig. 4 is a perspective view of one example of a culture vessel used in the automatic culture apparatus shown in Fig. 1.
Fig. 5 shows a side view of one example of a tip supplying apparatus used in the automatic culture apparatus shown in Fig. 1.
Fig. 6 is a perspective view of one example of a reagent supplying apparatus used in the automatic culture apparatus shown in Fig. 1.
Fig. 7 is a flowchart illustrating the operation of the culture treatment apparatus shown in Fig. 1.
Fig. 8 is a perspective view of an automatic culture apparatus according to another embodiment of the present invention.
Fig. 9 shows a processing flow of processing to be carried out in the event of a power failure while cells in a culture vessel are cultured using the automatic culture apparatus shown in Fig. 8.
Fig. 10 is a front elevational view of another example of a lid-opening/closing section used in the automatic culture apparatus shown in Fig. 8.

### Best Mode for Carrying Out the Invention

### First Embodiment

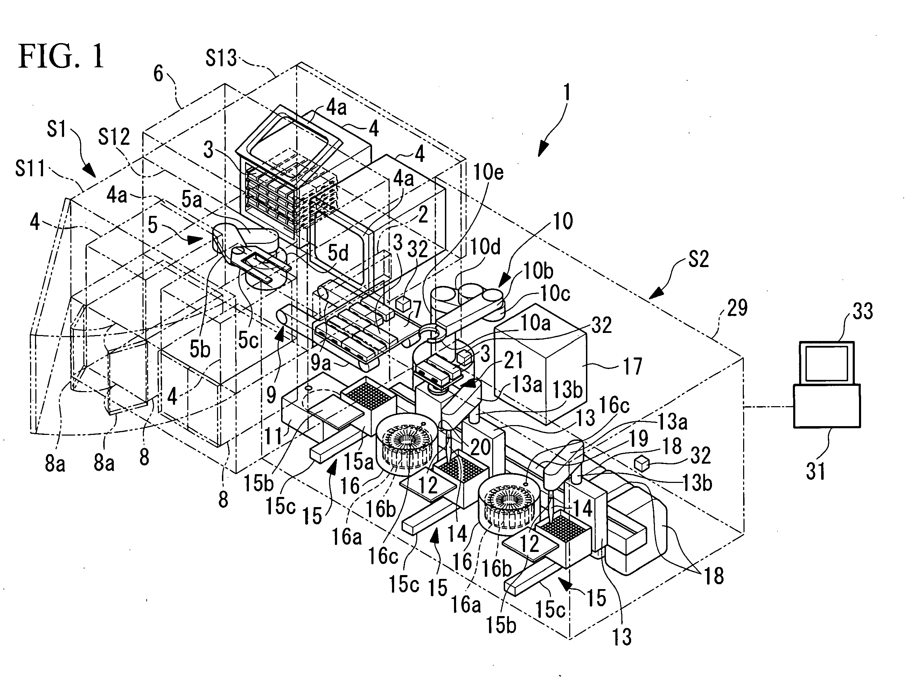

An automatic culture apparatus according to a first embodiment of the present invention will now be described with reference to Figs. 1 to 7.

An automatic culture apparatus 1 according to this embodiment is hermetically sealed with a transparent wall material that allows external observation and includes a first space S1 and a second space S2, which communicate with each other via a shutter 2.

Two incubation chambers 4, which hold culture vessels (containers for the automatic culture apparatus) 3, are disposed in each of side spaces S11 and S13 inside the first space S1 to make a total of four incubation chambers 4. A transfer robot (transfer mechanism) 5 for moving the culture vessels 3 is provided in a central space S12. At the upper part of the central space S12, an air cleaning unit 6 that sends a downward flow of clean air in order to clean the air inside the central space S12 is provided. By providing each of the four incubation chambers 4 with a door 4a facing the central space S12, two doors 4a arranged side-by-side face another two doors 4a, with a space disposed therebetween.

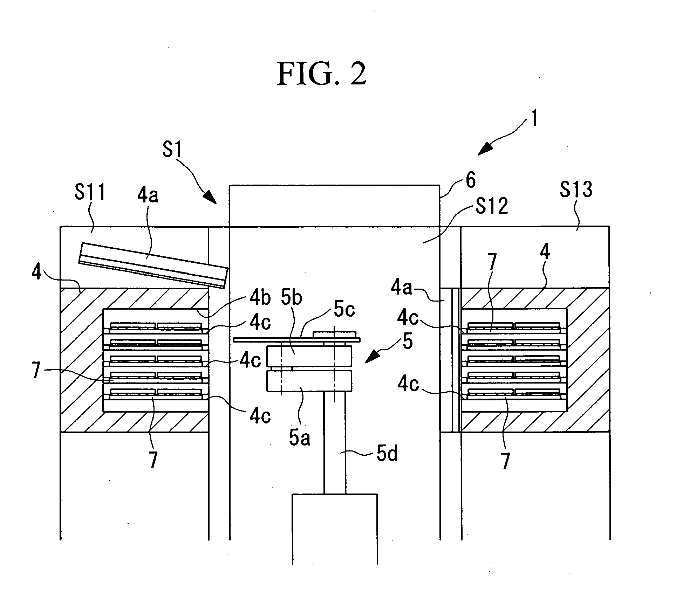

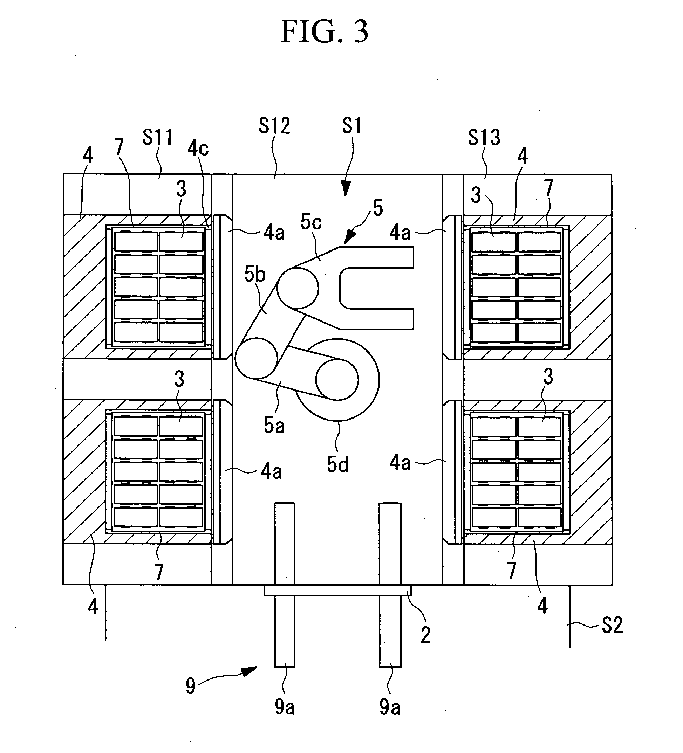

As shown in Figs. 2 and 3, each incubation chamber 4 has an opening 4b at one side thereof, and each opening 4b is provided with the door 4a, which can open and close at the opening 4b. In the side walls facing each other at the left and right sides of the opening 4b, a plurality of rail-shaped tray holding members 4c are provided at corresponding heights. By placing trays 7 across the pairs of tray holding members 4c at the left and right sides, it is possible to store trays 7 at a plurality of levels in the height direction. Predetermined incubation conditions are maintained inside each incubation chamber 4, for example a temperature of 37±0.5°C, a humidity of 100%, and a CO₂ concentration of 5%. The tray holding members are not limited to rail-shaped members; they may be formed in any shape so long as they allow the trays to be inserted and removed while being supported.

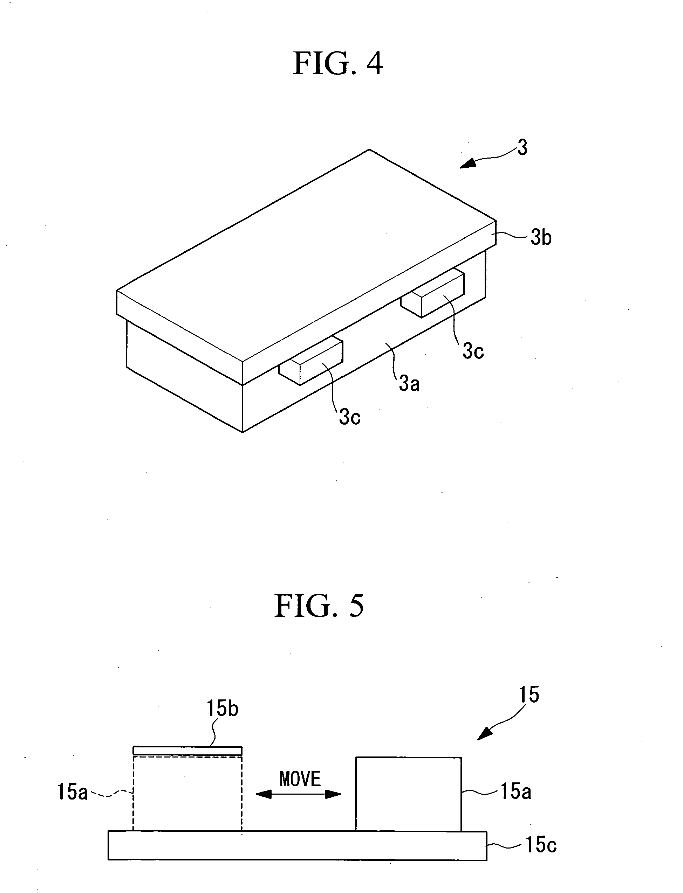

On each tray 7, a plurality of, for example ten, culture vessels 3 can be mounted side-by-side. As shown in Fig. 4, each culture vessel 3 includes a vessel main body 3a and a lid 3b provided at the top of the vessel main body 3a. On the left and right side surfaces of the vessel main body 3a, projections 3c to be gripped by a hand in the second space, to be describes below, are provided.

A stocker 8 that contains a plurality of unused culture vessels 3 mounted on trays 7 is disposed below each incubation chamber 4. Each stocker 8 has a door 8a that can be opened and closed at a side facing towards the outside of the first space S1 in the opposite direction to the door of the incubation chamber 4. The door 8a is formed with a size large enough to open one entire side of the stocker 8.

The above-described transfer robot 5 is arranged substantially in the center of the gap between the four incubation chambers 4. The transfer robot 5 includes a horizontally rotatable first arm 5a; a second arm 5b that is continuous with the end of the first arm 5a so as to be rotatable about a vertical axis; a hand 5c that is attached to the end of the second arm 5b so as to be rotatable about a vertical axis, and that does not have a mechanism that might degrade the environment inside the incubation chamber, such as a drive unit or power train; and a raising and lowering mechanism 5d that can raise and lower the first arm 5a, the second arm 5b, and the hand 5c. With this configuration, the transfer robot 5 can access all trays 7 inside the four incubation chambers 4, and in addition, it has a horizontal operating range such that it can pass trays 7 onto a conveyer 9 disposed between the first space S1 and the second space S2 via the shutter 2.

The conveyer 9 includes two endless belts 9a disposed at the left and right with a space therebetween larger than the width of the hand 5c of the transfer robot 5, so that the trays 7 can be placed across the endless belts 9a. In addition to the transfer robot 5 being able to access all trays 7 inside the incubation chambers 4, it also has a vertical-direction operating range such that it can access at least the uppermost tray 7 in the stocker 8.

The belts 9a are not limited to endless belts.

The hand 5c is formed in a flat shape extending horizontally so that the trays 7 can be mounted thereon, and is formed with a thickness that can be inserted into the space between trays 7 when stored in the incubation chambers 4. Thus, by raising the hand 5c from the position at which it is inserted into the space between the trays 7, the trays 7 can be pushed up from below by two prongs and removed from the tray holding members 4c, and in addition, the trays 7 can be held stably.

A culture treatment apparatus 29 is provided in the second space S2. This culture treatment apparatus 29 includes: a handling robot (treatment section) 10 for handling the culture vessels 3 on the tray 7 transferred from the first space S1 by the conveyer 9 with the shutter 2 opened; a centrifugal separator 11 for separating cells from the culture media in the culture vessels 3; two dispensing robots 13 each including an automatic pipette 12 for dispensing a variety of liquids, such as blood serum, reagents, and the like, and capable of horizontal rotation and upward and downward motion; three tip supplying apparatuses 15 storing a plurality of disposable tips 14 to be mounted at the ends of the automatic pipettes 12 of these dispensing robots 13 and capable of providing these tips 14 in the operating regions of the dispensing robots 13; tip recovery units (not shown in the figure) for recovering used tips 14 for disposal; reagent supplying apparatuses 16 for holding various liquids, such as blood serum and reagents, in a plurality of containers; a microscope 17 for allowing examination of the appearance of cells inside the culture vessels 3; a plurality of reservoir tanks 18 for holding respective waste solutions discarded after replacing the reagents and culture media; a horizontal transfer mechanism 19 for moving the culture vessels 3 so that the culture vessels 3 can be handed over between the conveyer 9 and the robots 10 and 13; and a shaker 21 that is mounted on a slider 20 of the horizontal transfer mechanism 19 and that places thereon a received culture vessel 3 to vibrate the culture vessel 3.

An air cleaning unit (not shown in the figure) producing a downward flow of clean air for cleaning the air inside the second space S2 is also provided inside the second space S2.

The above-described handling robot 10 is a horizontally articulated robot for horizontally moving, raising, and lowering a gripping hand 10a for handling the culture vessels 3. In the example shown in Fig. 1, the handling robot 10 includes three inter-linked horizontal arms 10b, 10c, and 10d, and a raising and lowering mechanism 10e for raising and lowering these horizontal arms 10b to 10d. At the ends of the horizontal arms 10b to 10d, in addition to the gripping hand 10a for gripping the culture vessels 3, an automatic pipette (not shown in the figure) that can attach and detach a tip 14 to supply cells and culture media in the culture vessel 3 and remove cells and culture media from the culture vessel 3, and a hand (not shown in the figure) for opening and closing a lid 3d by catching the lid 3b of a culture vessel 3 to open it are provided.

The handling robot 10 opens and closes the lid 3b of a culture vessel 3 on the tray 7 transferred with the conveyer 9, grips the culture vessel 3 to transport it to the shaker 21 and the microscope 17, replaces the tip 14 at the end of the automatic pipette, and places the cell-containing culture medium taken out of the culture vessel 3 into the centrifugal separator 11. Therefore, various devices including the conveyer 9, the shaker 21, the microscope 17, the tip supplying apparatuses 15, the tip recovery units (not shown in the figure), and the centrifugal separator 11 are disposed within the operating range of the handling robot 10.

Also, by rotating the cell-containing culture medium supplied from the handling robot 10 at low speed, the above-described centrifugal separator 11 separates cells with a high specific gravity suspended in the culture medium from the culture medium, and the separated cells settle.

The dispensing robots 13 each include a horizontally rotatable arm 13a having the automatic pipette 12 to which the tips 14 are removably attached at the end thereof, and a raising and lowering mechanism 13b for raising and lowering the arm 13a. The dispensing robots 13 supply culture medium or various reagents to the culture vessels 3 transferred by the horizontal transfer mechanism 19. Therefore, various devices, such as the shaker 21 on the horizontal transfer mechanism 19, the tip supplying apparatuses 15, the tip recovery units, and the reagent supply apparatuses 16, are disposed within the operating range of the dispensing robots 13.

As shown in Figs. 1 and 5, the tip supplying apparatuses 15 contain a plurality of the tips 14 in an array, with openings for attaching to the automatic pipette 12 facing upwards, inside containers 15a which are open at the top. The handling robot 10 and dispensing robots 13 are designed so that tips 14 are attached to the ends of the automatic pipette 12 simply by inserting the automatic pipette 12 from the top when new tips 14 are required. The containers 15a are attached to transfer mechanisms 15c so that they can be moved back and forth between lids 15b and the operating regions of the handling robot 10 and the dispensing robots 13. In order to prevent dust etc. from being deposited on the tips 14, the containers 15 are arranged below the lids 15b by operating the transfer mechanisms 15c, except when the tips 14 are to be replaced.

The above-described tip recovery apparatuses have gripping apparatuses for gripping the tips 14 at the inlets of recovery containers. When tips 14 used in the handling robot 10 and the dispensing robots 13 are inserted into the gripping apparatuses, the gripping apparatuses grip the tips 14. Thus, when the handling robot 10 and the dispensing robots 13 move the automatic pipettes 12 in this state, the used tips 14 are removed from the ends of the automatic pipettes 12, and are collected in the recovery containers.

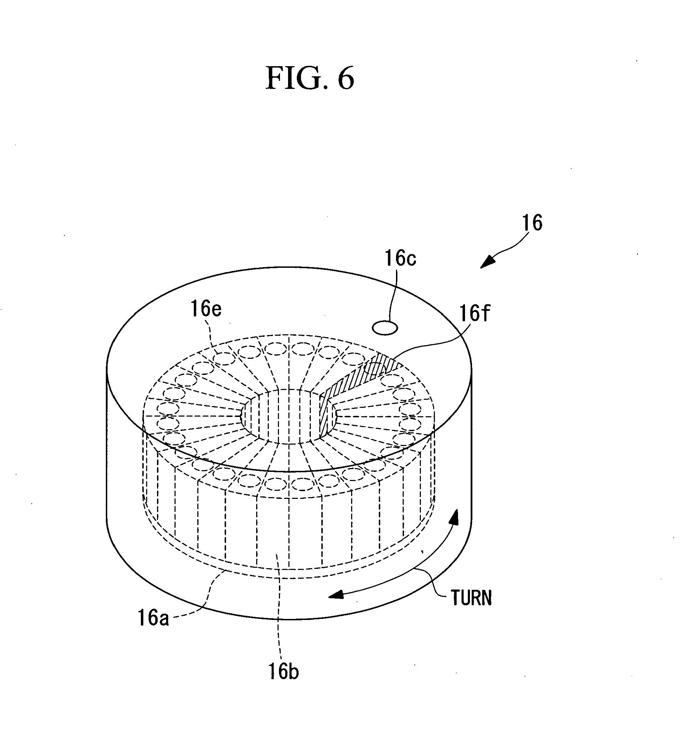

As shown in Figs. 1 and 6 for example, the reagent supplying apparatuses 16 each contain a horizontally rotatable table 16a inside a cylindrical casing, and a plurality of cylindrical reagent containers 16b having a fan-shaped bottom surface are mounted on the table 16a around the circumference thereof. Various reagents and so forth are held in reagent containers 16b. Examples include MEM (minimum essential medium) forming a necessary culture medium for culturing cells; DMEM (Dulbeccos's Modified Eagle Medium); serums such as FBS (Fetal Bovine Serum) or human serum; enzymes for breaking down proteins, such as trypsin, which release cells in the culture vessel 3; growth factor such as cytokine, which promotes cellular growth during incubation; differentiation-inducing factor such as dexamethasone, which promotes cell differentiation; antibiotics like penicillin-based antibiotics; hormones such as estrogen; and nutrients such as vitamins and so on.

Insertion holes 16c through which the dispensing robots 13 insert tips 14 at the ends of the automatic pipettes 12 are provided in the top surfaces of the casings of the reagent supplying apparatuses 16. These insertion holes 16c are disposed within the operating range of the dispensing robots 13. Also, each of the reagent containers 16b has an opening 16e at the upper surface thereof, which is disposed at a position corresponding to the position of the insertion hole 16c. With this arrangement, by rotating the tables 16a to position the openings e of the reagent containers 16b directly below the insertion holes 16c in the casings, the dispensing robots 13 insert the tips 14 at the ends of the automatic pipettes 12 into the reagent containers 16b from above, which allows the reagents etc. contained inside to be drawn up. In addition, at least one of the plurality of reagent containers 16b is an empty container 16f that contains no reagents. If it is not necessary to draw up, for example, reagents with the dispensing robots 13, each table 16a is rotated to position the empty container 16f directly below the opening 16c of the casing to prevent dust etc. from being deposited on the reagents. The empty container 16f is not limited to a container; the empty container 16f may be something like a block that closes the opening 16c. Two reagent supplying apparatus 16 and two dispensing robots 13 are provided in order to separately handle chemical solutions in the specimen, such as common trypsin, and liquids inherent to the specimen, such as blood serum.

The microscope 17 is used to examine the appearance of cells or the degree of proliferation inside the culture vessel 3 during the culturing process and when replacing the culture medium. In addition, the microscope 17 is used to count the number of cells. The microscope 17 is designed so that operations such as actuating-distance adjustment of an XY stage, changing the magnification, and so on can all be carried out remotely. An eyepiece may be disposed outside of the second space S2 to allow the condition of cells inside the culture vessels 3 to be visually examined from outside the automatic culture apparatus 1.

The reservoir tanks 18 contain DMEM, PBS (phosphor buffered saline), and so on, which can be commonly used in all specimens, and these liquids are supplied to the reagent containers 16a in the reagent supplying apparatuses 16 as required. The reservoir tanks 18 also include a tank serving as a waste tank for storing waste culture medium that is discharged when replacing the culture medium.

The horizontal transfer mechanism 19 includes the slider 20, which can be moved horizontally by means of a linear motion mechanism. The shaker 21 is mounted on the slider 20, and the culture vessel 3 mounted on the shaker 21 can be transferred from the conveyor 9 to the operating region of the dispensing robots 13.

The shaker 21 includes a holding mechanism (not shown in the figure) for holding the culture vessel 3 transferred from the tray 7 on the conveyer 9 and an agitator (not shown in the figure) for vibrating the culture vessel 3. For example, a device that oscillates the culture vessel 3 back and forth within a predetermined angular range may be employed as the agitator. A device that applies ultrasonic vibrations or a device that applies vibrations in the horizontal direction may be employed as the agitator.

A control apparatus 31 is connected to each of the devices in the automatic culture apparatus 1 according to this embodiment. The control apparatus 31 controls the sequence and timing of the various steps and also keeps a record of the operating history of the devices, and so on.

The culture treatment apparatus 29 further includes a plurality of particle counters 32 in the second space S2. Since the plurality of particle counters 32 are arranged in key positions in the second space S2, even a local decrease in the degree of cleanliness at a certain position in the second space S2 can be detected.

The particle counters 32 are connected to the above-described control apparatus 31. A threshold for the degree of cleanliness is stored in the control apparatus 31, and the stored threshold is compared with the degree of cleanliness detected by each particle counter 32. If the detected degree of cleanliness is below the threshold, the following processing is carried out. The control apparatus 31 stores, for example, class 100 as the threshold for the degree of cleanliness, so that if the degree of cleanliness detected by a particle counter 32 is below class 100, e.g., class 150, the following processing is carried out.

That is to say, the control apparatus 31 monitors the operation of each device of the culture treatment apparatus 29. For example, the control apparatus 31 monitors whether the hand of the handling robot 10 for opening and closing a lid 3b has opened the lid 3b of a culture vessel 3. Then, if a decrease in the degree of cleanliness at any position in the second space S2 is detected with a detection signal from any particle counter 32 while the control apparatus 31 detects that the lid 3b of a culture vessel 3 is open, the control apparatus 31 operates a program for closing the lid 3b of the culture vessel 3. More specifically, an interrupt is generated during normal program operation to continue the operation until the lid 3b of the culture vessel 3 is closed and then stop the operation.

The control apparatus 31 may be provided with a monitor 33 for displaying the degree of cleanliness in the second space S2 detected by each particle counter 32. With this monitor 33 provided, whether the interior of the second space S2 is clean can easily be checked from outside the second space S2.

The operation of the culture treatment apparatus 29 and the automatic culture apparatus 1 according to this embodiment, having the above configuration, will be described below.

In order to culture cells using the automatic culture apparatus 1 according to this embodiment, first, bone-marrow fluids taken from a patient are filled into a centrifuge receptacle (not shown) and are placed in the centrifugal separator 11 in the culture treatment apparatus 29. This step may be executed by an operator or with the handling robot 10. Then, by operating the centrifugal separator 11, bone marrow cells having a high specific density can be collected from the bone marrow.

The collected bone marrow cells are then placed in the culture vessels 3 by the handling robot 10. At this point, by operating the conveyor 9, ten empty culture vessels 3 mounted on the tray 7 are delivered from the first space S1 to the second space S2 with the lids 3b closed. After opening the two lids 3b in the delivered culture vessel 3, the handling robot 10 operates the gripping hand 10a to grip the culture vessel 3 and transfers it onto the shaker 21. A separate robot for opening the lids 3b may be provided. In this manner, the lids 3b can be opened just before treatment. This reduces the chance of foreign matter entering the vessel main body 3a.

When unused tips 14 are disposed within the operating region of the handling robot 10 by operating the transfer mechanism 15c of the tip supplying apparatus 15, the handling robot 10 receives an unused tip 14 from the tip supplying apparatus 15 and attaches the tip 14 to the end of the automatic pipette.

In this state, the handling robot 10 is operated to bring the tip 14 at the end of the automatic pipette 12 into contact with the bone marrow cells collected in the centrifugal separator 11. Then, by operating the automatic pipette 12, the bone marrow cells are drawn up into the tip 14. By operating the handling robot 10, the drawn-up bone marrow cells are filled into the culture vessel 3, with the lid 3d open, on the shaker 21.

Upon completion of filling the bone marrow cells into the culture vessel 3, the handling robot 10 transfers the tip 14 to the tip recovery unit to remove the tip 14. In addition, the tip supplying apparatus 15 arranges the container 15a below the lid 15b by operating the transfer mechanism 15c.

Next, by operating the horizontal transfer mechanism 19, the culture vessel 3 containing bone marrow cells is transferred horizontally together with the shaker 21, and is positioned within the operating region of the dispensing robots 13. By operating the automatic pipette 12 having an unused tip 14 received from the tip supplying apparatus 15 attached to the end thereof, the dispensing robot 13 draws up a suitable amount of DMEM, blood serum, or various reagents from the reagent container 16b in the reagent supplying apparatus 16, and thereafter transfers the tip 14 to above the culture vessel 3 to dispense the drawn-up liquid into the culture vessel 3. By rotating the table 16a at this time, the reagent container 16b is positioned directly below the opening 16c.

When drawing up the blood serum and reagents, the tip 14 is replaced with an unused tip 14 from the tip supplying apparatus 15 each time the reagents etc. are drawn up. By doing so, bone marrow cells in a suitable culture medium exist in a mixed state in the culture vessel 3. To uniformly distribute the bone marrow cells in the culture medium, the shaker 21 may be operated to vibrate together with the culture vessel 3. Then, when all treatment is completed, the culture vessel 3 is returned in the operating region of the handling robot 10 through the operation of the horizontal transfer mechanism 19. In this case, the reagent supplying apparatus 16 rotates the table 16a to position the empty container 16f directly below the opening 16c. The handling robot 10 places the lid 3b over the culture vessel main body 3a and returns the culture vessel 3 onto the tray 7.

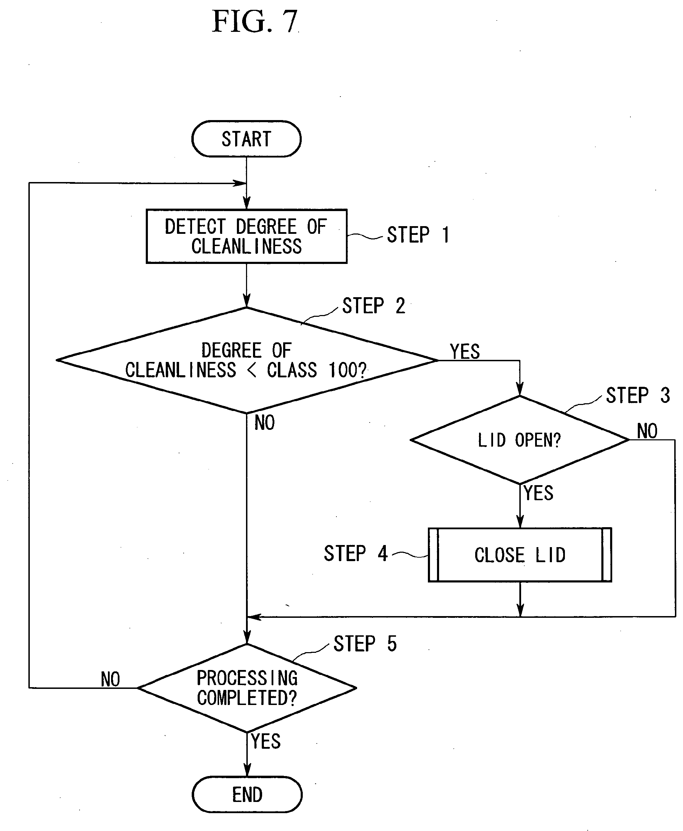

In this case, with the automatic culture apparatus 1 according to this embodiment, the plurality of particle counters 32 arranged in the second space S2 always count the degree of cleanliness in the second space S2 by counting particles, as shown in Fig. 7 (step 1). Then, if the degree of cleanliness detected by any particle counter 32 is below class 100 (step 2), it is determined whether the lid 3b of a culture vessel 3 is open through the operation of the control apparatus 31 (step 3) . Since the control apparatus 31 manages the operation of each device including the handling robot 10, it is determined whether the lid 3b of a culture vessel 3 is open according to the progress of the operating program.

Then, if it is determined by the control apparatus 31 that the lid 3b of a culture vessel 3 is open, the operating program is terminated or the completion of the operating program is awaited depending on the progress of the operating program. Thereafter, the control passes to a subroutine program for closing the lid 3b of the culture vessel 3 (step 4). In the subroutine program, an operating program for closing the lid 3b of the culture vessel 3 by operating, for example, the handling robot 10 is executed to close the lid 3b of the culture vessel 3. In this manner, if the degree of cleanliness in the second space S2 is below class 100, the lid 3b of the culture vessel 3 is closed. This reduces the risk of dust entering the culture vessel.

On the other hand, if it is determined in step 2 that the degree of cleanliness is above class 100 and in step 3 that the lid 3b of the culture vessel 3 is closed, it is determined in step 5 whether a series of operating programs have been completed, and the above-described processing is repeated while the operating programs are continued.

By operating the conveyer 9 after the culture treatment apparatus 29 has completed predetermined treatment for all culture vessels 3 on the tray 7, the culture vessels 3 mounted on the tray 7 are moved from the second space S2 into the central space S12 of the first space S1. By operating the transfer robot 5 in this state, the tray 7 is lifted by the hand 5c. Then, once it has been transferred to a point in front of the incubation chamber 4 for storing that tray 7, the door 4a of the incubation chamber 4 is opened, and the tray 7 is placed onto empty-tray holding members 4c using the transfer robot 5. Then, once the door 4a is closed, the incubation conditions inside the incubation chamber 4 are again kept constant, which allows the cells to be cultured.

In the same way as described above, when replacing the culture medium and the container, by operating the transfer robot 5, which is disposed outside the incubation chamber 4, the culture vessels 3 inside the incubation chamber 4 are taken out together with the tray 7 and are delivered from the first space S1 to the second space S2. In the second space S2, trypsin is dispensed into the culture vessels 3, and once cells inside the culture vessel 3 are released, they are placed in the centrifugal separator 11 by operating the handling robot 10, and only the required part, such as mesenchymal stem cells, is collected. The other processing steps are similar to those described above.

Then, by carrying out a culturing process for a predetermined period through a plurality of culture medium replacements and container replacements, the mesenchymal stem cells multiply to produce a sufficient number of cells. To determine whether or not a sufficient number of cells have been produced, the culture vessel 3 having the mesenchymal stem cells adhering to the bottom surface is transferred to the microscope 17 by operating the handling robot 10, and examination or measurement is carried out to determine the degree of proliferation of the cells. The culture vessels 3 mounted on the tray 7 may contain the same types of specimen, or culture vessels 3 containing different specimens may be mixed. The culture vessels 3 mounted on the shaker 21 may contain the same types of specimen, or culture vessels 3 containing different specimens may be mixed.

By doing so, with the automatic culture apparatus 1 according to this embodiment, it is possible to automatically culture a desired number of mesenchymal stem cells from bone marrow taken from a patient.

With the automatic culture apparatus 1 according to this embodiment, no mechanical parts for removing the culture vessels 3 exist inside the incubation chambers 4. In other words, only the tray holding members 4c for supporting the trays 7 are provided inside the incubation chambers 4 and the mechanical parts for removing the culture vessels 3 are completely integrated in the transfer robot 5, which is disposed outside the incubation chambers 4. Thus, after completing extraction and insertion of the trays 7, the transfer robot 5 can remain entirely outside the door 4a of the incubation chambers 4.

Therefore, when the doors 4a are closed, no mechanical parts exist inside the incubation chambers 4, and therefore, no dust is produced inside the chambers, such as the kind of dust normally produced when operating mechanical parts. In addition, although a temperature of 37±0.5°C, a humidity of 100%, a CO₂ concentration of 5%, and so forth are maintained inside the incubation chambers 4, since there are no mechanical parts, there are no problems such as corrosion, despite these conditions. Furthermore, even if the doors 4a are opened, since only the end of the hand 5c of the transfer robot 5 enters the incubation chambers 4, no rotating mechanism or sliding mechanism actually enters the incubation chambers 4. Therefore, dust is prevented from entering the incubation chambers 4 to maintain the degree of cleanliness inside the incubation chambers 4.

Since the automatic culture apparatus 1 according to this embodiment is provided with the air cleaning unit 6 at the top of the central space S12 where the transfer robot 5 is disposed, the degree of cleanliness in the central space S12 where the transfer robot 5 is disposed can always be maintained. Therefore, even when the doors 4a of the incubation chambers 4 are opened, it is possible to minimize the amount of dust entering the incubation chambers 4.

Therefore, with the automatic culture apparatus 1 according to this embodiment, the risk of contamination of the cells being cultivated due to dust or the like is reduced, which affords an advantage in that stable and effective cell culturing can be realized.

In the above-described first embodiment, if it is determined by a particle counter 32 that the degree of cleanliness in the second space S2 is below a threshold, the control apparatus 31 is operated to check the open/closed state of the lid 3b of a culture vessel 3 and close the lid 3b. Alternatively, the particle counters 32 may check the degree of cleanliness only when the lid 3b of a culture vessel 3 is open. Furthermore, if the lid 3b of the culture vessel 3 is closed when it is determined by the particle counter 32 that the degree of cleanliness detected is below the threshold, the operating program may be stopped at a point just before the next opening of the lid 3b. If the degree of cleanliness in the second space S2 is low, instead of closing the lid 3b of the culture vessel 3, the container 15a of each tip supplying apparatus 15 may be moved below the lid 15b or the reagent containers 16b of each reagent supplying apparatus 16 may be positioned away from below the insertion hole 16c.

The particle counters 32 may be located at any positions as long as the degree of cleanliness in the second space S2 can be detected. In particular, in order to detect the degree of cleanliness near the culture vessels 3, it is preferable that the particle counters 32 be located near the path through which the culture vessels 3 pass. It is further preferable that a particle counter 32 be arranged on the shaker 21 for holding a culture vessel 3 thereon.

### Second Embodiment

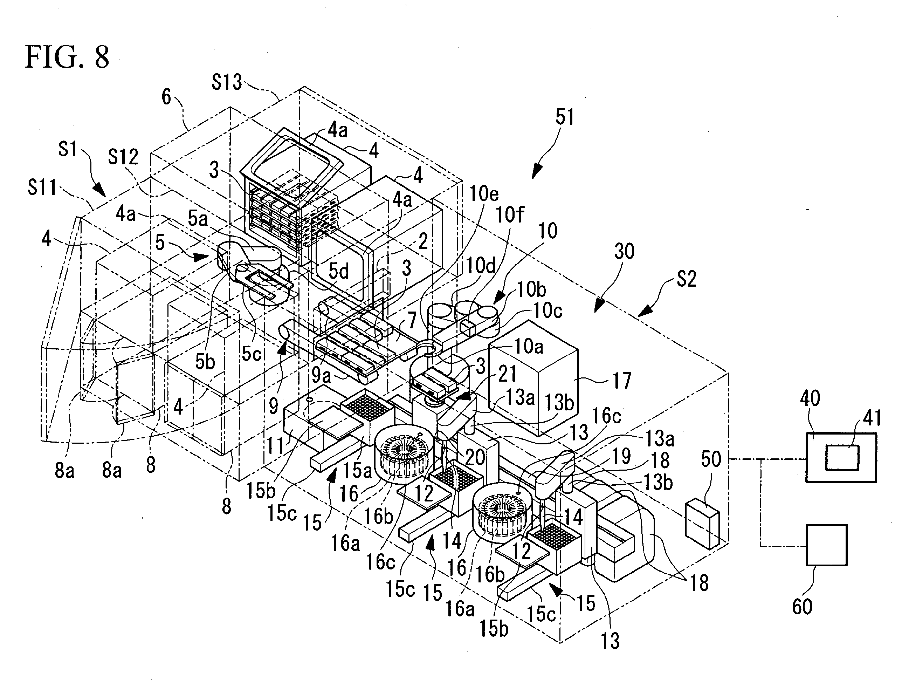

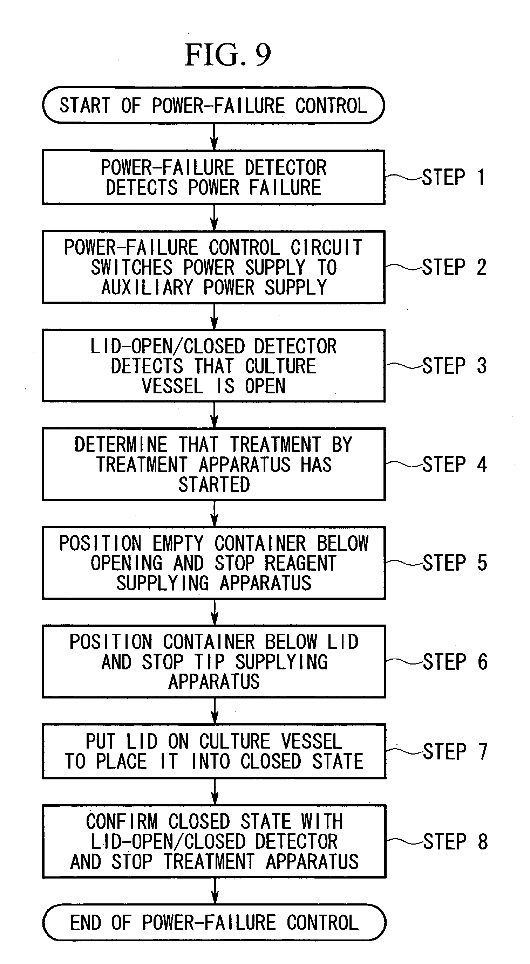

An automatic culture apparatus according to a second embodiment of the present invention will now be described with reference to Figs. 2 to 6 and Figs. 8 and 9. The same components as those used in the automatic culture apparatus according to the first embodiment are denoted by the same reference numerals, and thus will not be described in detail.

An automatic culture apparatus 51 according to this embodiment is hermetically sealed with a transparent wall material that allows external observation, like the automatic culture apparatus 1 according to the first embodiment, and includes a first space S1 and a second space S2, which communicate with each other via a shutter 2.

The structure in the first space S1 is the same as that according to the first embodiment.

On the other hand, a culture treatment apparatus 30 is disposed in the second space S2 of the automatic culture apparatus 51 according to this embodiment. This culture treatment apparatus 30 includes a control apparatus 40 for controlling individual devices in the incubation chambers 4 and the culture treatment apparatus 30; a power-failure detector (power-failure detecting section) 50 for detecting a power failure; and an auxiliary power supply 60 for supplying electrical power in the event of a power failure.

The culture treatment apparatus 30 includes a handling robot (treatment section) 10 for handling the culture vessels 3 on the tray 7 transferred from the first space S1 by the conveyer 9 with the shutter 2 opened and for opening/closing lids 3b of the culture vessels 3; a centrifugal separator 11 for separating cells from the culture media in the culture vessels 3; two dispensing robots 13 each including an automatic pipette 12 for dispensing a variety of liquids, such as blood serum, reagents, and the like, and capable of horizontal rotation and upward and downward motion; three tip supplying apparatuses 15 storing a plurality of disposable tips 14 to be mounted at the ends of the automatic pipettes 12 of these dispensing robots 13 and capable of providing these tips 14 in the operating regions of the dispensing robots 13; tip recovery units (not shown in the figure) for recovering used tips 14 for disposal; reagent supplying apparatuses 16 for holding various liquids such as blood serum and reagents in a plurality of containers; a microscope 17 for allowing examination of the appearance of cells inside the culture vessels 3; a plurality of reservoir tanks 18 for holding respective waste solutions discarded after replacing the reagents and culture media; a horizontal transfer mechanism 19 for moving the culture vessels 3 so that the culture vessels 3 can be handed over between the conveyer 9 and the robots 10 and 13; and a shaker 21 that is mounted on a slider 20 of the horizontal transfer mechanism 19 and on which a received culture vessel 3 is placed to vibrate the culture vessel 3.

An air cleaning unit (not shown in the figure) producing a downward flow of clean air for cleaning the air inside the second space S2 is also provided inside the second space S2.

As with the first embodiment, the handling robot 10 includes three horizontal arms 10b, 10c, and 10d linked to one another and a raising and lowering mechanism 10e for raising and lowering these horizontal arms 10b to 10d. The horizontal arm 10b has therein a lid-open/closed detector (lid-open/closed detecting section) 10f for monitoring the gripping hand 10a to detect the opening/closing of the lid 3b of the culture vessel 3. By doing so, the culture vessel 3 can be changed from a closed state, where the lid 3b is closed, to an open state, where the lid 3b is opened.

The above-described power-failure detector 50 is a device such as a relay interposed in a main power circuit not shown in the figure. It has a mechanism that operates if the main power supply is shut down due to, for example, a power failure to notify a power failure control circuit (power-failure control section) 41, to be described below, of the power failure.

The control apparatus 40 controls the overall sequence and timing of the various steps of not only the incubation chambers 4 and the culture treatment apparatus 30 described above, but also other components. The control apparatus 40 also keeps a record of the operating history etc. In addition, the control apparatus 40 has a power-failure control circuit 41 that switches the power supply to the auxiliary power supply 60 if the power-failure detector 50 detects a power failure and that operates the handling robot 10 to place the lid 3b of a culture vessel 3 into a closed state if the lid-open/closed detector 10f detects that the lid 3b of the culture vessel 3 is in an open state.

Furthermore, this power-failure control circuit 41 has a function that, if treatment by the culture treatment apparatus 30 has already started when a power failure is detected by the power-failure detector 50, allows the treatment to continue until the subsequent closing of the lid 3b of the culture vessel 3 and then stops the culture treatment apparatus 30. In other words, the power-failure control circuit 41 has a function for controlling the culture treatment apparatus 30 in the event of power failure.

The auxiliary power supply 60 contains a supply power, such as a battery, with a function for supplying electrical power at least until the culture treatment apparatus 30 completes predetermined treatment on a culture vessel 3 in an open state once a power failure has occurred.

A cell culturing process using the automatic culture apparatus 51 with the above-described structure will be described below.

The automatic culture apparatus 51 according to this embodiment operates in the same manner as the automatic culture apparatus 1 according to the second embodiment as long as treatment of the cells in a culture vessel is normally carried out, and thus, a description thereof for normal operation will be omitted.

A case where a power failure occurs when, for example, the dispensing robot 13 is approaching the reagent supplying apparatus 16 to draw in reagents etc. while the cells in the culture vessel 3 are being treated will be described with reference to Figs. 5, 6, 8, and 9.

First, the power-failure detector 50 detects the occurrence of a power failure as a result of the main power supply being shut down and outputs a detection result to the power-failure control circuit 41 of the control apparatus 40 (step 1). When a detection result is input from the power-failure detector 50, the power-failure control circuit 41 switches the power supply to supply electrical power from the auxiliary power supply 60 (step 2). Furthermore, the lid-open/closed detector 10f of the handling robot 10 detects that the lid 3b of a culture vessel 3 is in an open state and inputs the detection result to the power-failure control circuit 41 (step 3). As soon as a detection result is input from the lid-open/closed detector 10f, the power-failure control circuit 41 recognizes that the dispensing robot 13 and the reagent supplying apparatus 16 are operating. In short, the power-failure control circuit 41 determines that the culture treatment apparatus 30 has started predetermined treatment (step 4).

Then, the power-failure control circuit 41 allows the dispensing robot 13 to continue to operate, so that the dispensing robot 13 draws in reagents etc. from the reagent supplying apparatus 16 and dispenses the reagents etc. into the culture vessel 3. By rotating the table 16a of the reagent supplying apparatus 16 after completing supply of the reagents etc., the empty container 16f is positioned directly below the opening 16c of the casing and the reagent supplying apparatus 16 is stopped (step 5). By doing this, dust etc. is prevented from being deposited on the reagents etc. in the casing in the event of a power failure.

Furthermore, the power-failure control circuit 41 causes the tip supplying apparatus 15 to continue to operate until predetermined treatment is completed. More specifically, after the tip supplying apparatus 15 has supplied a tip 14 to the automatic pipette 12 of the dispensing robot 13, the transfer mechanism 15c is controlled to position the container 15a below the lid 15b and the tip supplying apparatus 15 is then stopped (step 6). By doing this, dust etc. is prevented from being deposited on unused tips 14 stored in the container 15a in the event of a power failure.

Further, when predetermined treatment of the cells in the culture vessel 3 is completed, the power-failure control circuit 41 moves the slider 20 of the horizontal transfer mechanism 19 to position the culture vessel 3 in the operating region of the handling robot 10. Next, the gripping arm 10a of the handling robot 10 is operated to cover the culture vessel 3 with the lid 3b (step 7) . Then, after confirming that the culture vessel 3 is covered with the lid 3b, that is, the culture vessel 3 is in a closed state, with the lid-open/closed detector 10f, the power-failure control circuit 41 stops the horizontal transfer mechanism 19 and the handling robot 10 (step 8) . In short, the power-failure control circuit 41 stops the culture treatment apparatus 30 after allowing the culture treatment apparatus 30 to complete predetermined treatment. By doing this, dust etc. is prevented from being deposited on the cells in the culture vessel 3 in the event of a power failure.

As described above, in this automatic culture apparatus 51, even if a power failure occurs while reagents etc. required to culture cells are being dispensed, the power-failure control circuit 41 switches the power supply to the auxiliary power supply 60 based on detection information from the power-failure detector 50. This ensures a power supply for continuing operation. Furthermore, in response to the lid-open/closed detector 10f having detected an open state of the lid 3b of the culture vessel 3, the power-failure control circuit 41 operates the handling robot 10 to close the lid 3b of the culture vessel 3 to place it in a closed state. This prevents dust etc. from being deposited on the cells in the culture vessel 3.

Furthermore, monitoring of culture vessels 3 by an operator, which was previously required, is now unnecessary. This reduces the time and cost required for monitoring.

Furthermore, since the culture vessels 3 are in a closed state, a procedure for recovering from a power failure can be carried out safely even if the recovery procedure requires each part to be checked.

Furthermore, if the culture treatment apparatus 30 is performing predetermined treatment when a power failure occurs, the power-failure control circuit 41 stops the culture treatment apparatus 30 after the culture treatment apparatus 30 has advanced the predetermined treatment. Therefore, the culture treatment apparatus 30 does not experience premature termination of treatment. In short, dust etc. is prevented from being deposited on the reagents etc. in the reagent supplying apparatus 16 and on unused tips 14 in the tip supplying apparatuses 15. Thus, in short, disposal of contaminated products can be prevented, and excessive use of disposable products can be eliminated. Furthermore, since the culture treatment apparatus 30 only has to carry out normal processing, it is not necessary to prepare a separate processing procedure for power failure.

A case where a power failure occurs when reagents etc. are dispensed into the cells in a culture vessel 3 has been described in the above-described embodiment. If a power failure occurs, for example, before the lid of a culture vessel 3 transferred with the conveyer 9 is opened with the handling robot 10, the culture treatment apparatus 30 stops operating.

In the above-described embodiment, the culture treatment apparatus 30 is stopped after the culture vessel 3 is covered with the lid 3b using the handling robot 10. Alternatively, the culture treatment apparatus 30 may be stopped, for example, when the culture vessel 3 is returned onto the tray 7 and stored in an incubation chamber 4.

Although the lid-open/closed detecting section is employed in this configuration, it is also acceptable that the open/closed state of the lid be determined through programming, for example, a CPU, instead of using the lid-open/closed detecting section. In this case, it is needless to say that the CPU is backed up in the event of a power failure.

Since the automatic culture apparatus 51 according to this embodiment is provided with the air cleaning unit 6 at the top of the central space S12 where the transfer robot 5 is disposed, the degree of cleanliness in the central space S12 where the transfer robot 5 is disposed can always be maintained. Therefore, even when the doors 4a of the incubation chambers 4 are opened, it is possible to minimize the amount of dust entering the incubation chambers 4.

Therefore, with the automatic culture apparatus 51 according to this embodiment, the risk of contamination of the cells being cultured due to dust or the like is reduced, which affords an advantage in that stable and effective cell culturing can be realized.

The present invention is not limited to the structures described in the first and second embodiments. That is to say, the configuration and number of the incubation chambers 4, the transfer robot 5, the handling robot 10, the dispensing robots 13, and various other devices are not limited, but may be set as desired according to the application.

In the above-described embodiment, the handling robot 10 is employed as the treatment section for opening and closing a lid. Alternatively, for example, a robot that exclusively opens and closes a lid may be provided.

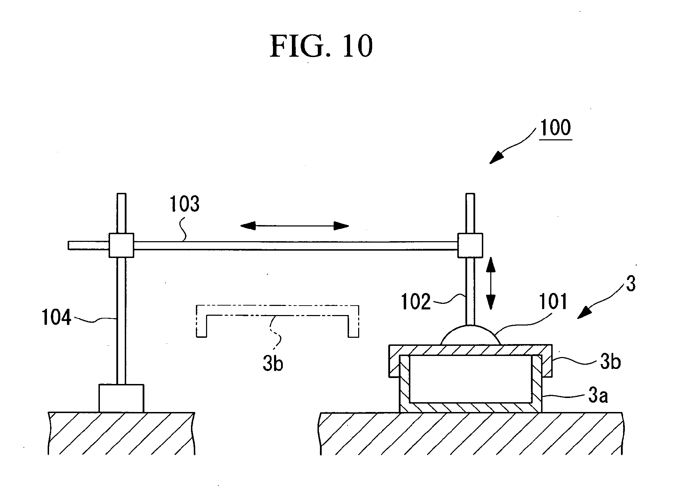

For example, a lid-opening/closing robot 100, as shown in Fig. 10, may be used. This lid-opening/closing robot 100 includes a suction plate 101 that can apply suction to the lid 3b of the culture vessel 3; a vertical arm 102 that can move the suction plate 101 in the vertical direction; a horizontal arm 103 that can move the vertical arm 102 in the horizontal direction; and a support arm 104 for supporting the horizontal arm 103. In this lid-opening/closing robot 100, the horizontal arm 103 and the vertical arm 102 are operated to position the suction plate 101 on the top surface of the lid 3b. Next, the horizontal arm 103 and the vertical arm 102 can be operated with the lid 3b being sucked by the suction plate 101 to open the lid 3b of the culture vessel 3, which is thereby placed in an open state. In addition, the culture vessel 3 is placed in a closed state by reversing the procedure described above. Although in the second embodiment, the lid-open/closed detector (lid-open/closed detecting section) 10f is provided in the horizontal arm 10b of the handling robot 10, the lid-open/closed detecting section may be provided on the lid-opening/closing robot 100.

The incubation chambers 4 may be any type of incubator used for culturing, such as CO₂ incubators, multi-gas incubators, standard incubators, or refrigerated incubators. Alternatively, they may be a combination of any of the above.

In addition, the applications, operating procedure, operating conditions, etc. for the culture treatment apparatus and the automatic culture apparatus according to the present invention are not limited to those described in the above-described first and second embodiments.

In the above-described first and second embodiments, a culture vessel is employed as the container for the automatic culture apparatus. Alternatively, a reagent container or a disposable-tip container may be employed. If a reagent container is adopted as the container for the automatic culture apparatus, dust etc. is prevented from being deposited on reagents. If a disposable-tip container is adopted, dust etc. is prevented from being deposited on tips.

In the above-described first and second embodiments, a lid is used as a member for closing the container for the automatic culture apparatus (culture vessel) . The member for closing the container is not limited to a lid, but any type of closing member can be employed to close the opening of the container.

In the above-described embodiment, cytokine is used as an example of growth factor. Instead of cytokine, any material that encourages growth may be used as the growth factor, including, for example, platelet concentrate, BMP, EGF, FGF, TGF-β, IGF, PDGF, VEGF, HGF or a combination thereof. Furthermore, although a penicillin-based antibiotic has been described as an antibiotic in the above-described embodiment, any antibiotic may be employed, including cephems, macrolides, tetracyclines, fosfomycins, aminoglycosides, new quinolones, and so on, instead of a penicillin-based antibiotic.

It goes without saying that the order in which the bone marrow cells, DMEM, blood serum, and various reagents are filled or drawn up may be changed as desired.

The automatic culture apparatus according to the present invention is not limited to the purpose of culturing bone-marrow mesenchymal stem cells. Cells taken from various tissues or established cell lines may be cultured.

After obtaining a sufficient number of mesenchymal stem cells using the automatic culture apparatus according to the present invention, a tissue filling material such as calcium phosphate and a differentiation-inducing factor such as dexamethasone are introduced into the culture vessels, and by repeating the culturing process, a tissue filling material that can compensate for tissue deficiencies may be produced. If cells are to be cultured while being attached to a tissue filling material, instead of calcium phosphate, any material having compatibility with tissue, preferably bioabsorbable material, may be used, as the tissue filling material. In particular, porous ceramic having biocompatability, collagen, polylactic acid, polyglycolic acid, hyaluronic acid, or any combination of these may be used. In addition, a metal like titanium may be used. Furthermore, the tissue filling material may be in granular form or in bulk form.

### Industrial Applicability

According to the culture treatment apparatus and the automatic culture apparatus of the present invention, even if there is a change in the state of a space where cells stored in a container, such as a culture vessel, for the automatic culture apparatus are subjected to predetermined treatment, quick and appropriate action can be taken.

If a cleanliness sensor is employed as the detection section in the culture treatment apparatus, the culture treatment apparatus and the automatic culture apparatus according to the present invention effectively reduce the amount of dust etc. entering the container, such as a culture vessel, for the automatic culture apparatus to prevent contamination of the cells, which allows stable cells to be automatically cultured stably.

In addition, if the culture treatment apparatus is provided with an auxiliary power supply and a power-failure control section, in the event of a power failure, the power-failure control section switches the power supply to the auxiliary power supply based on detection information from the power-failure detecting section and closes the lid of the container, such as a culture vessel, for the automatic culture apparatus before stopping the culture treatment apparatus. Therefore, the culture treatment apparatus and the automatic culture apparatus according to the present invention can prevent dust etc. from being deposited on the cells in the container, which may be caused by premature termination in the event of a power failure. Furthermore, monitoring in the event of a power failure, which was previously required, is now unnecessary. Therefore, the time and cost required for monitoring can be reduced.

## Claims

1. A culture treatment apparatus comprising:
a treatment section for applying predetermined treatment to cells stored in an openable container for an automatic culture apparatus in a partitioned space;
a detection section for detecting a predetermined state of the space, the detection section being in the space or in the vicinity of the space; and
a control section for controlling the treatment section to prevent the container in the space from being opened if the detection section detects the predetermined state.

2. A culture treatment apparatus comprising:
a treatment section for applying predetermined treatment to cells stored in an openable container for an automatic culture apparatus in a partitioned space;
a detection section for detecting a predetermined state of the space, the detection section being in the space or in the vicinity of the space; and
a control section for controlling the treatment section to close the container if the detection section detects the predetermined state with the container opened.

3. The culture treatment apparatus according to claim 1 or 2, wherein the detection section is a cleanliness sensor for detecting whether a degree of cleanliness in the space does not satisfy a predetermined degree of cleanliness.

4. The culture treatment apparatus according to claim 3, further comprising a report section for reporting that the degree of cleanliness measured with the cleanliness sensor does not satisfy the predetermined degree of cleanliness, if the degree of cleanliness measured with the cleanliness sensor does not satisfy the predetermined degree of cleanliness.

5. The culture treatment apparatus according to claim 3, wherein a plurality of the cleanliness sensors is arranged at intervals in the space.

6. The culture treatment apparatus according to claim 3, wherein the cleanliness sensor is arranged near a location through which the container passes.

7. The culture treatment apparatus according to claim 6, wherein the cleanliness sensor is arranged on a mounting stage for holding the container.

8. The culture treatment apparatus according to claim 3, further comprising a display section for displaying the degree of cleanliness measured with the cleanliness sensor.

9. The culture treatment apparatus according to claim 2, further comprising:
an auxiliary power supply for supplying electrical power in the event of a power failure, wherein
the detection section is a power-failure detecting section for detecting a power failure, and
the control section switches power supply to the auxiliary power supply if the power-failure detecting section detects a power failure.

10. The culture treatment apparatus according to claim 9, wherein, if the predetermined treatment has started when a power failure is detected by the power-failure detecting section, the control section allows the predetermined treatment to be continued until the subsequent closing of the container for the automatic culture apparatus and stops the culture treatment apparatus.

11. The culture treatment apparatus according to claim 9, further comprising an open/closed detecting section for detecting an open/closed state of the container for the automatic culture apparatus.

12. The culture treatment apparatus according to claim 1 or 2, wherein the container for the automatic culture apparatus is at least one of a culture vessel containing cells, a reagent container, and a disposable-tip container.

13. An automatic culture apparatus comprising:
the culture treatment apparatus according to claim 1 or 2;
an incubation chamber for accommodating the container storing cells such that the container can be extracted and inserted and for culturing the cells while maintaining predetermined incubation conditions; and
a transfer mechanism for transferring the container between the culture treatment apparatus and the incubation chamber.
